# EUROPEAN PATENT APPLICATION

(11) **EP 1 253 426 A1**
(43) Date of publication of application: **30.10.2002**
(21) Application number: 01901366.3
(22) Date of filing: 17.01.2001
(51) Int. Cl.: G01N 33/53, G01N 33/68

(54) **METHOD FOR EXAMINATION OF BRONCHIAL ASTHMA ATTACK AND EXAMINATION KIT**

(30) Priority: 18.01.2000 JP 2000009281
(71) Applicant: Projectyou Co., Ltd., Tsukuba-shi, Ibaraki 305-0024 (JP)
(72) Inventor: UCHIDA, Yoshiyuki, Tsukuba-shi, Ibaraki 305-0032 (JP)
(74) Representative: Melzer, Wolfgang, Dipl.-Ing.
(86) International application number: JP0100240
(87) International publication number: WO01053832

(57) **Abstract**

The method for examination of bronchial asthma attack in the invention is characterized in that it is based on the amount of the E-cadherin decomposition product being present in a sample to be tested. According to the invention, it becomes possible to ascertain whether or not attack occurs or what stage the pathological state of an asthmatic is, by utilizing the E-cadherin decomposition product as a marker and examining how much the E-cadherin decomposition product is contained in a sample to be tested. This allows providing an appropriate therapy or treatment depending on the pathological state.

The determination of the amount of the E-cadherin decomposition product being present in a sample to be tested is preferably carried out according to an immunological technique using an antibody reactive to the E-cadherin decomposition product in view of simplicity, etc.

According to the invention, a kit for examination of bronchial asthma attack based on the amount of the E-cadherin decomposition product being present in a sample to be tested, the kit comprising an antibody reactive to the E-cadherin decomposition product, is provided.

## Description

### Technical Field

The present invention relates to a method for examination of bronchial asthma attack and an examination kit, reflecting the pathological state.

### Background Art

It is well known that bronchial asthma, regarded as one of the present-day diseases, has been much interested since the number of the patients goes on increasing. It is difficult to predict the attack of bronchial asthma. In clinical fields, accordingly, it is necessary to ascertain the pathological stage of a patient to give an appropriate therapy or treatment depending on his pathological state. At present, in monitoring of the patient, a peak-flow meter monitoring the degree of bronchial obstruction has been used. It has been desired, however, to provide a method for examination of the asthma in a convenient manner using a sample to be tested, and reflecting the pathological state.

In this situation, recent year, bronchial asthma has been defined as a chronic airway inflammatory disorder, and in such a view efforts have been focused on elucidation of its mechanism.

On the other hand, it is known that E-cadherin is a membrane-permeable type of Ca²⁺-dependent glycoprotein expressed primarily on epithelial cells and acts as adhesion molecule between epithelial cells. From the results of a variety of studies, it has been reported that normal expression and the functional activity of E-cadherin are critical for the maintenance of tight junctions between epithelial cells and for maintaining normal function of the paracellular barrier.

Therefore, though the relationship between bronchial asthma and E-cadherin expressed on the airway epithelial cells is much interested, there are no reports in this regard.

The purpose of the invention is to provide a method for examination of bronchial asthma attack and an examination kit, reflecting the pathological state.

### Disclosure of the Invention

In view of the above-described facts, the present inventor has studied in great detail bronchial asthma from a viewpoint of chronic airway inflammatory disorder. As a result, he found that in bronchial asthma attack E-cadherin expressed on the airway epithelial cells is decomposed and the decomposition product is released from the cells to trigger another attack making the pathological state worse.

The present invention was made based on the above findings. The method for examination of bronchial asthma attack in the invention, as described in Claim 1, is characterized in that the examination is carried out on the basis of the amount of the E-cadherin decomposition product being present in a sample to be tested.

In Claim 2, there is provided the method for examination as claimed in Claim 1, wherein the E-cadherin decomposition product is soluble E-cadherin.

In Claim 3, there is provided the method for examination as claimed in Claim 1, wherein the sample to be tested is sputum.

In Claim 4, there is provided the method for examination as claimed in Claim 1, wherein the examination is carried out in an immunological technique using an antibody reactive to the E-cadherin decomposition product.

In the invention, a kit for examination of bronchial asthma attack, as described in Claim 5, comprises at least one of antibodies reactive to the E-cadherin decomposition product to examine bronchial asthma attack on the basis of the amount of the E-cadherin decomposition product being present in a sample to be tested.

### Best Mode for Carrying Out the Invention

The method of the invention for examination of bronchial asthma attack is characterized in that the examination is carried out on the basis of the amount of the E-cadherin decomposition product in a sample to be tested. As mentioned above, during bronchial asthma attack, E-cadherin expressed on the airway epithelial cells is decomposed and the decomposition product is released from the cells. Therefore, in healthy persons or even in asthmatics if they have no symptoms of the attack, the E-cadherin decomposition product released from the cells in the subject's samples could not be detected at all or hardly detected. On the contrary, occurrence of bronchial asthma attack increases the content of the decomposition product. By utilizing the E-cadherin decomposition product as a marker for detection and determining it in a sample to be tested, it becomes possible to confirm the severity of the pathological state of a patient, permitting an appropriate therapy or treatment according to the state, accordingly.

In the invention, by the E-cadherin decomposition product is meant proteins or peptides derived from E-cadherin contained in a sample to be tested, released from the airway epithelial cells during bronchial asthma attack, in which the major decomposition product is soluble E-cadherin. Soluble E-cadherin is an extracellular portion of E-cadherin, a protein with the molecular weight of approximately 85 kDa. Decomposition of E-cadherin is considered due to proteolysis caused by a variety of factors derived from inflammatory cells such as eosinophils.

As for the samples to be tested, there is no limitation as far as they can be collected in *in vitro* diagnostic treatment, and for example, sputum (including induced sputum), airway lavaged fluids, serum, plasma, urine, and the like are included, with sputum being desirable in view of easiness of preparation of the sample to be tested and reduction of the burden of a patient. The sputum may be used directly as a sample or as a supernatant obtained by centrifugal separation.

As for the methods for detection of the amount of the E-cadherin decomposition product in a sample to be tested, there is no limitation, but immunological techniques using an antibody or antibodies reactive to the E-cadherin decomposition product are preferably used because they are convenient. Such a method includes but is not limited particularly to enzyme immunoassay, radioimmunoassy, fluoroimmunoassay, immune nephelometry, latex agglutination, Western blot analysis, etc. Any of these methods may be carried out according to a convenient procedure in the respective methods.

The antibody or antibodies reactive to the E-cadherin decomposition product may be either polyclonal or monoclonal ones. In raising the antibodies, a conventionally used animal such as mouse, rat, rabbit, and the like, may be used without any inconvenience. For these antibodies, a person skilled in the art may optionally perform an operation such as fragmentation, labeling, immobilization, modification, and the like, in a conventional technique. As for practically used antibodies, a monoclonal antibody ECCD-2 derived from a rat, monoclonal antibody HECD-1 or SHE78-7 derived from mice (they are available from Takara Shuzo), and the like, each having specific reactivity against soluble E-cadherin, are exemplified.

For example, using ECCD-2 and HECD-1 which respectively recognize different antigen-determining sites, one is adsorbed on a solid phase such as microplate, and the other is labeled with an enzyme peroxidase; thus, a sandwich type of enzyme immunoassay system can be constituted. In this case, in addition to ECCD-2 and HECD-1, a variety of auxiliary agents conventionally used in an immunological technique, such as a substrate for measuring an enzymatic activity, reaction terminator, washing agent, and the like, may be used together to yield a kit, which may conventionally be used in examination of bronchial asthma attack.

In order to confirm the severity of the pathological state of a patient from the amount of the E-cadherin decomposition product in a sample to be tested, for example, a calibration curve may be made for detection of the amount of the E-cadherin decomposition product in the sample and compared with the values of healthy subjects. In such a case, in depicting a calibration curve, if required it may be corrected with albumin in the sample to be tested.

### Example

In the following example, the method of the invention for examination of bronchial asthma attack will be explained in detail, but the following description is not intended to limit the invention.

### (Experimental Method)

### 1. Preparation of Guinea-pig Asthma Models

The models were prepared according to the procedure as reported by the present inventors (Uchida, Y. et al., J. Pharmacol. Exp. Ther. 277: 1622-1629, 1996). Briefly, the preparation was carried out as follows. Female Hartley guinea pigs, weighing 250 to 300 g (SLC Farm) were pretreated with 30 mg/kg of cyclophosphamide intraperitoneally. Two days later, the animals were sensitized to 1 mg of ovalbumin (OVA) emulsified in 100 mg of aluminum hydroxide by intraperitoneal injection. Three weeks after the sensitization, 10 µg of OVA emulsified in 100 mg of aluminum hydroxide were intraperitoneally injected as a booster. Three weeks after the booster injection, these guinea pigs were challenged with inhalation of OVA (2 mg/ml in saline) as an antigen to induce asthma attack, and they are used as asthma models.

### 2. Measurement of Paracellular Permeability

The guinea pigs as asthma models were assigned to four groups (before the antigen challenge, 30 minutes, 3 hours and 6 hours after the antigen challenge) (three animals per group) and treated as follows.

Before the antigen challenge, 30 minutes, 3 hours and 6 hours after the antigen challenge, guinea pigs were anesthetized by intraperitoneal injection of 50 mg/kg of pentobarbital, then killed by exsanguination, and treated according to the procedure described by Rnaga et al. (Rnaga, V. et al., Am. Res. Respir. Dis. 28: 1065-1070, 1983). Briefly, the procedure was as follows. A solution of 2.5 mg of horseradish peroxidase (HRP) (Sigma; type IV) dissolved in 0.5 ml of phosphate-buffered physiological saline (PBS)(pH 7.4) was instilled into the upper portion of the tracheas using a 27G needle attached to a 1 ml syringe. After 5 minutes, the tracheal segments distal to the site of instillation were removed and fixed by immersion in 0.1 M phosphate buffer (PB) (pH 7.4) containing 2.0% glutaraldehyde. Thereafter, each was cut longitudinally into three pieces and rinsed thoroughly with PB. HRP was visualized by treating the tissues for 30 minutes with diaminobenzidine (Sigma; 3,3'-diaminobenzidine tetrahydrochloride; Grade II)(DAB)(Dojindo) in 0.1 M Tris-hydrochloric acid buffer (pH 7.2) containing 0.01% hydrogen peroxide.

Tissues were rinsed again with PB, fixed for 1 hour with 1% aqueous osmium tetroxide, dehydrated in alcohol and then in propylene oxide, and embedded in Epon resin (trade name)(DuPont). Semi-thin sections of 1 µm in thickness were cut with a glass knife and stained with to luidine blue. Proximal intercellular spaces numbered 1,000 or more per section. These spaces were counted under a light microscope. Paracellular permeability was expressed as the percentage of the penetrated intercellular spaces of HRP. Ultra-thin sections of 100 nm in thickness, cut with a diamond knife by an Ultrotome III (LKB), were observed with an H-7000 electron microscope (Hitachi).

### 3. Immunohistologic Analysis

In the same manner as described above, before the challenge, 30 minutes and 6 hours after the challenge, guinea pigs were killed. Their tracheas were immediately removed and fixed with 10% phosphate-buffered formalin (Wako Pure Chemical Industries) for 2 hours. The resulting samples were washed with PBS, incubated in 5%, 10% and 20% sucrose-containing PBS, and then frozen. Air-dried cryostat sections of 10 µm in thickness were rehydrated by washing with a buffer (0.5 M sodium chloride, 0.02 M Tris, 0.01 M calcium chloride, 0.1% Tween 20, pH 7.4) for 5 minutes and incubated for 30 minutes with 0.3% hydrogen peroxide-containing methanol to block endogenous peroxidase. The resulting samples were blocked for 1 hour with a mixture of normal goat serum (Dako Japan Co., Ltd.) and Dako Protein Block Serum-Free (trade name) (Dako Japan Co., Ltd.), followed by washing with a buffer.

For detection of soluble E-cadherin, a rat's monoclonal antibody ECCD-2 (Takara Shuzo) specific to the extracellular portion of E-cadherin was used as a primary antibody. Sections with the antibody were incubated for 30 minutes at room temperature. After washing three times with a buffer, the sections were incubated with a biotinylated goat anti-rat secondary antibody (Organon Teknica Corp.), followed by incubation with an avidin-biotin-peroxidase complex (Vector Laboratories).

Sites of immunoreaction were visualized by immersing the sections in a solution of DAB and hydrogen peroxide.

For electron microscopic analysis, the sections were fixed for 2 hours with 2.5% glutaraldehyde in 0.1 M sodium phosphate buffer (pH 7.2), followed by the reaction with DAB. Thereafter, the sections were fixed with 2% aqueous osmium tetroxide for 4 hours, then dehydrated with ethanol (50% - 100%) followed by propylene oxide, and embedded in Poly/Bed 812 resin (Polysciences, Inc.). Ultra-thin sections of 150 nm in thickness, cut with a diamond knife by an Ultrotome LKB 2088 (LKB-Produkter AB), were observed with an H-7000 electron microscope.

### 4. Western Blot Analysis

In the same manner as described above, before the antigen challenge, 1 hour, 3 hours and 6 hours after the antigen challenge, guinea pigs were killed, and their tracheas were immediately removed. The luminal side of the removed tracheas was washed with 0.5 ml of ice-cold PBS containing a cocktail of protease inhibitors (trade name: Complete) (Boehringer Mannheim) to collect the tracheal lavaged fluids. The lavaged fluids were centrifuged at 400g for 5 minutes at 4°C and then concentrated ten-fold by using Centricon 10 (trade name)(Millipore Corp.). The concentrated fluid was mixed with an equal volume of loading buffer (125 mM Tris-hydrochloric acid, pH 6.8; 4% sodium dodecylsulfate; 20% glycerol; 0.05% bromophenol blue; 5% β-mercaptoethanol) and boiled for 3 minutes. The resulting samples were subjected to SDS-polyacrylamide gel electrophoresis on an acrylamide gel (Bio-Rad Laboratories). After electrophoresis, the gels were blotted onto a PVDF membrane (Bio-Rad Laboratories). The gel-blotted PVDF membranes were blocked for 1 hour with 5% dry milk powder in a buffer and then incubated with ECCD-2 for 30 minutes at room temperature. After three washes with buffer, the membranes were incubated with a biotinylated goat anti-rat secondary antibody, followed by incubation with an avidin-biotin peroxidase complex.

### (Experimental Result)

### 1. Paracellular Permeability

In measurement of paracellular permeability, few HRP reactions were seen in the intercellular spaces before the antigen challenge. DAB deposits were observed extensively in the intercellular spaces 30 minutes and 6 hours after the challenge, but not generally 3 hours after the challenge. The epithelium 30 minutes and 3 hours after the challenge showed narrow intercellular spaces. On the other hand, the intercellular spaces in the epithelium were wider than the above case 6 hours after the challenge of antigen.

The penetration of HRP in the intercellular spaces was observed 30 minutes after the challenge but it decreased rapidly 3 hours after the challenge; the penetration was again increased 6 hours after the challenge.

### 2. Localization of E-Cadherin in Tracheal Epithelium

In immunohistological analysis, the immunoreactivity of E-cadherin was widely diffused in the cytoplasm before the challenge and 30 minutes after the challenge of antigen. Particularly, it was localized at the lateral membranes and apicolateral border of the epithelial cells where adherence junctions are located. However, the immunoreactivity of the lateral membranes of the epithelial cells decreased 6 hours after the challenge of antigen.

### 3. Detection of Soluble E-Cadherin in Lavage Fluid of the Trachea

Since the immunohistochemical analysis revealed that immunoreactivity of E-cadherin in adherence junctions decreased 6 hours after the challenge, as described above, soluble E-cadherin in the lavaged fluid of the trachea was examined by the Western blot analysis. As a result, soluble E-cadherin with a molecular weight of 85 kDa was detected 6 hours after the antigen challenge, though it was not detected before the challenge, and 1 hour and 3 hours after the challenge.

### (Conclusion)

It was revealed that 6 hours after the challenge of antigen E-cadherin being present in the airway epithelial cells is decomposed and its extracellular portion, soluble E-cadherin, is released from the cells into the tracheal lavaged fluids. At that time, the intercellular spaces of the airway epithelium were considered to be widened to increase permeability of HRP; the reason is that decomposition of E-cadherin might cause damage of the intercellular adherence function.

Bronchial asthma attack can roughly be classified into immediate airway response and late airway response, the former being observed immediately after the antigen challenge and the latter about 6 hours after the antigen challenge. In human, the late response has been regarded as more important. According to the above-described experiment, in the late response, it was elucidated that the extracellular portion of E-cadherin was cleaved to exude moisture into the airway lumen, that decomposition of E-cadherin widened the intercellular spaces, and that the cleaved soluble E-cadherin was increased in the airway.

In fact, the present inventor confirmed that the sputum of patients suffering from bronchial asthma attack contain a greater amount of soluble E-cadherin than that of healthy persons, and that the value increases with a change of pathological state for the worse. Thus, utilizing this phenomenon in a diagnostic field, it becomes possible to ascertain whether or not attack occurs or what stage the pathological state of an asthmatic is, by examining how much soluble E-cadherin is contained in a sample to be tested, such as sputum or tracheal lavaged fluids.

### Industrial Applicability

According to the invention, it becomes possible to ascertain whether or not attack occurs or what stage the pathological state of an asthmatic is, by utilizing the E-cadherin decomposition product as a marker and examining how much the E-cadherin decomposition product is contained in a sample to be tested, and to give an appropriate therapy or treatment depending on the pathological state.

## Claims

1. A method for examination of bronchial asthma attack, which is **characterized in that** the examination is carried out on the basis of the amount of the E-cadherin decomposition product being present in a sample to be tested.

2. The method for examination as claimed in Claim 1, wherein the E-cadherin decomposition product is soluble E-cadherin.

3. The method for examination as claimed in Claim 1, wherein the sample to be tested is sputum.

4. The method for examination as claimed in Claim 1, wherein the examination is carried out in an immunological technique using an antibody reactive to the E-cadherin decomposition product.

5. A kit for examination of bronchial asthma attack, which comprises at least one of antibodies reactive to the E-cadherin decomposition product to examine bronchial asthma attack on the basis of the amount of the E-cadherin decomposition product being present in a sample to be tested.
